# EUROPEAN PATENT SPECIFICATION

(11) **EP 0 695 421 B1**
(45) Date of publication and mention of the grant of the patent: **27.06.2001**
(21) Application number: 94915410.8
(22) Date of filing: 25.04.1994
(51) Int. Cl.: G01N 21/76, G01N 33/53

(54) **METHOD OF MEASURING CHEMILUMINESCENCE OF MULTIPLE SAMPLES ON A CONTINUOUS MATRIX**
CHEMIILUMINESZENZ VON MEHRFACHPROBEN AUF EINER KONTINUIERLICHEN MATRIX
PROCEDE DE MESURE DE LA CHIMIOLUMINESCENCE DE PLUSIEURS ECHANTILLONS PLACES SUR UNE MATRICE CONTINUE

(30) Priority: 23.04.1993 FI 931858
(43) Date of publication of application: 07.02.1996
(73) Proprietor: POTTER, Colin Gerald, Headington, Oxford, Oxon 0X3 7RR (GB); ANDERSON, Larry J., Atlanta, GA 30333 (US)
(72) Inventor: POTTER, Colin Gerald, Headington, Oxford, Oxon 0X3 7RR (GB); ANDERSON, Larry J., Atlanta, GA 30333 (US)
(74) Representative: Abrams, Michael John
(86) International application number: US9404471
(87) International publication number: WO9425855

(56) References cited:
- EP-A- 0 046 563
- EP-A- 0 194 132

## Description

### FIELD OF THE INVENTION

This invention relates to a method of and apparatus for detecting chemiluminescence from a multiplicity of discrete samples arrayed on a surface in a defined format, such as a matrix.

### BACKGROUND OF THE INVENTION

Various assay systems including immunoassays, receptor-ligand assays and probe hybridization are known for detecting desired chemical and biological information. For example, probe hybridization is used to detect specific nucleic acid sequences in a sample and involves the formation of a base-paired duplex from two single stranded nucleic acid molecules. With probe hybridization, in the dot-blot technique, the nucleic acid may be bound to a flat matrix such as a nylon membrane or nitrocellulose paper and detected by reacting it against a labelled nucleic acid that is complementary to a portion of the bound nucleic acid. Such labeled nucleic acid probes can be used to detect target sequences by hybridization to DNA or RNA.

The most commonly used label in assay systems for nucleic acids is radioactive phosphorus (³²P). Although radioactive labels are widely used in research laboratories, well-known problems with such radioactive labels - e.g., short half-life, safety and disposal problems, and the necessity to perform long auto-radiographic exposures for detection - generally preclude their use in clinical and other settings. This has resulted in the development of a number of non-radioactive labels, and associated detection methods. For example, probes labelled with fluorescein and with enzymes such as alkaline phosphatase and peroxidase are in common use. However, utilization of these labels has been limited by their lack of sensitivity compared with ³²P.

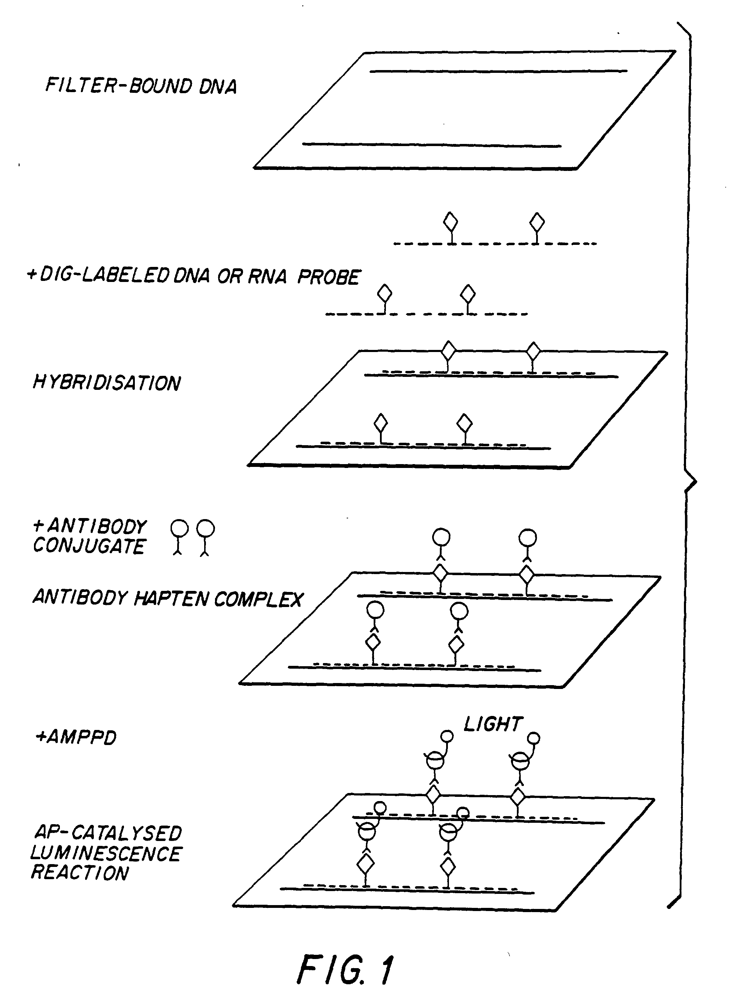

Recently, non-radioactive labeling and detection systems for nucleic acids have been introduced based on detection of chemiluminescence which have sensitivities closer to that of ³²P. Noteworthy among such chemiluminescence detection systems are: Enhanced Chemiluminescence (ECL) from Amersham International; Flash™ from Stratagene Cloning Systems; and Digoxygenin (DIG) from Boehringer Mannheim. FIG. 1 illustrates the hybridization and chemiluminescence process for the DIG system.

As is well known, chemiluminescence is a form of luminescence resulting from chemical reactions. (The reaction leading to light emission in bioluminescence is similar but generally requires either oxygen or hydrogen peroxide as a reactant.) With either chemiluminescence or bioluminescence, light is emitted in the form of single photons. Other forms of luminescence are generally distinguished by the form of stimulus for the light emission, e.g., fluorescence, where light is emitted in response to an external stimuli, such as light, and scintillation, where multiple photons are produced by a single event, such as an electron emitted from a decaying radioactive atom.

Currently, in probe hybridization assays, chemiluminescence is most often detected by exposing the matrix containing the sample plus hybridized labelled probe to X-ray film for a period of 10 min. to several hours, depending on the required sensitivity. Such autoradiography does not permit direct quantification of chemiluminescence, but densitometry may be applied to the x-ray film to obtain a quantitative measure of limited range.

An alternative approach is to use photodetectors to read the chemiluminescence. A common instrument having such detectors is the liquid scintillation counter. Such counters normally operate in a coincidence mode, in which a pair of oppositely disposed photo-multiplier tubes must both detect an event for the event to be registered as a muld-photon event characteristic of scintillation. However, by turning off one of such oppositely-disposed photo-multiplier tubes, the machine can count single photon events. In this manner, a liquid scintillation counter could be used to detect and quantify chemiluminescence, essentially through a measure of the number of photons emitted over a defined time interval.

For DNA and other work, chemiluminescent samples may be disposed as sample spots bound to a membrane, such as nylon, that could be separated by cutting them out and placing in scintillation vials for counting. This separation step is a serious disadvantage, precluding application of the method to a large number of samples. While detection of chemiluminescence and other light emission from multi-welled plate assays are well known, special instrumentation is necessary and such instruments measure the electric current produced in a photodetector by the incident chemiluminescent light. Alternatively, it is proposed here to quantify the chemiluminescence from samples, preferably, multiple samples on an intact matrix produced by, for example, digoxygenin-labelled DNA probes, using a flat-bed multi-detector scintillation counter with one set of oppositely-disposed photo-multiplier (PM) tubes disabled. (Such a flat- bed scintillation counter is described in US Patent No. 4,298,796 and UK Patent nt No. 1 586 966, issued to Warner & Potter.) With this technique, the limitation that separate samples must be placed in separate scintillation vials for chemiluminescent counting is avoided.

It is known from US 4,283,490 to provide a luminescence scintillation counter having a high-pass optical filter. This filter is used to increase signal to noise ratios, but is if no utility in addressing the oversensitivity of standard counters.

US 4,277,437 discloses a kit for performing a chemiluminescence assay using a light-emitting reciprocal pair of ligand and antiligand.

US 4,978,614 discloses an assay method directed towards the reaction with an enzyme of a specific dioxetane including a gel electrophoresis step.

EP 0 194 132 discloses an imaging immunoasssay detection system and method for detecting and quantifying multiple light-emitting reactions from small volume samples simultaneously.

### SUMMARY OF THE INVENTION

An object of the present invention is an improved method of counting chemiluminescence using photodetectors.

A principal object of the present invention is a method of counting multiple chemiluminescent samples that are not separated but are arranged as an array on a continuous support matrix.

In order to accomplish the above, a method and apparatus are proposed for assay systems that use chemiluminescence as a detection means, e.g., systems for detecting specific nucleic acid sequences and other macromolecules using a non-radioactively labeled ligand or probe hybridization to produce chemiluminescence and wherein samples to be tested are not separated but are instead arranged as an array on a continuous matrix and detection of such chemiluminescence is accomplished by photodetector-based instrumentation. Optical filters having transmission characteristics determined in accordance with the luminance to be detected are disposed between the sample and the photodetector. The method and apparatus according to the present invention also can be used for double chemiluminescent labelling or radioactive and chemiluminescent labelling as will be described.

According to a first aspect, the present invention provides a method for performing a chemiluminescent assay comprising: depositing a test sample on a supporting matrix; effectuating chemiluminescence of the test sample; filtering light radiating from the test sample as a result of said chemiluminescence; and counting chemiluminescence from the filtered light; characterised in that said filtration step comprises using a neutral density light attenuation optical filter to remove low energy bursts of light giving rise to high count rates of low amplitude pulses.

According to a second aspect, the present invention provides an apparatus for performing a chemiluminescence assay on multiple test samples deposited together on a continuous support, the continuous support containing a reactant that effectuates chemiluminescence of the multiple test samples, the apparatus comprising: flat bed means for holding the multiple test samples deposited on the continuous support during the performance of the assay; light detector means for detecting light emitted during chemiluminescence of the multiple test samples, said light detector means being disposed opposite said flat bed; chemiluminescence counter means for counting chemiluminescence of the multiple test samples, said chemiluminescence counter means coupled to said light detector means; and filter means for filtering light emitted during chemiluminescence and passing the light to said light detector means, said filter means being disposed in the optical path between said flat bed means and said light detector means; characterised in that said filter means is a neutral density light attenuation optical filter that precludes low energy bursts of light giving rise to high count rates of low amplitude pulses from being detectable by said light detector means.

According to a third aspect, the present invention provides a method of conducting electrophoresis comprising the steps of: depositing multiple test samples on a continuous supporting matrix; conducting gel electrophoresis; effectuating chemiluminescence of the test samples for a suitable exposure time; filtering light radiating from the multiple test samples as a result of the chemiluminescence with a neutral density filter; and counting chemiluminescence from the filtered light; characterised in that said filtration step comprises using a neutral density light attenuation optical filter to remove low energy bursts of light giving rise to high count rates of low amplitude pulses.

### BRIEF DESCRIPTION OF THE DRAWINGS

FIG. 1 shows the hybridization and chemiluminescence process for the DIG system from Boehringer Mannheim.

FIGS. 2A and 2B show an embodiment of the system of the invention

FIGS. 3A and 3B show sample pulse height spectrum for positive and background samples counted without filtration.

FIGS. 3C and 3D show noise pulse height spectrum for positive and background samples counted with filtration.

FIG. 4A shows the relationship between mean pulse height channel, SQP(I), and filter density.

FIG. 4B shows the relationship between count rate and filter density for neutral density filtration disposed between the sample and the PM tube.

FIGS. 5A-D show the effect of cross-talk using the method of the invention as measured over a 5-day period.

FIG. 6 shows typical results for the method of the invention using a 6-tube flat bed counter designed for 96 well plates or microtitration format filter matrices.

FIG. 7 illustrates normalized transmittance characteristics for colored filters usable for the double labeling embodiment of the invention.

FIGS. 8 and 9 show pulse characteristics for ³²P labeled probes in a dual radioactive/chemiluminescent labeling mode.

FIGS. 10 and 11 show pulse characteristics for chemiluminescent labeled probes in a dual radioactive/chemiluminescent labeling mode.

### DETAILED DESCRIPTION OF THE INVENTION

As a test bed for development of the disclosed invention, oligonucleotide probes were used for hybridization to samples derived from PCR (Polymerase Chain Reaction, Cetus Corp.) that were spotted on a nylon transfer membrane (Boehringer) and hybridized according to the method recommended by Boehringer (See Boehringer Mannheim Biochemica technical bulletins entitled *DIG Ollgonucleotide 3'-End Labeling Kit* (Cat. No. 1362372) and *DIG Luminescent Detection Kit* (Cat. No.13653514)). The chemiluminescent reagent AMPPD® was added, which acts as a substrate for alkaline phosphatase conjugated to a FAB antibody that recognizes digoxin-labelled oligonucleotide probes (FIG. 1). The nylon filters were wrapped, while still wet, in plastic film (e.g., Saran wrap) and placed between the perforated plates of a cassette and thence placed in position for counting by a multidetector counter.

One of the problems which has been associated with the attempted use of a multi-channd scintillation counter to count chemiluminescence is that the light output needed for maximum signal-to-noise ratio is so great that some counting registers in the multichannel analyzer of the counter, particularly those used for counting at the lowest end of the pulse height spectrum, become filled almost instantly. For a multi-detector system this resulted in very few counts detected for some samples and, although it gives high count rates, the precision could be very poor.

This problem can be explained by the fact that it is thought that chemiluminescence is often emitted in bursts of light, when considered on a microsecond basis, giving rise to high count rates of low amplitude pulses in the photomultiplier (PM) tube. For the multiple heads of such a multi-detector system, when attempting to count at a very high rate, one of the PM tubes will by chance receive such a high total count in one of the registers of its multichannel analyzer that it must stop counting, which in turn causes the other PM tubes to also turn Off. The counting time might have been only a few microseconds, such that when the count rate per minute is calculated, it may be very high. In another PM tube, however, the sample being counted may have been relatively inactive during the short interval of the count, and it is possible that only one or two events may have been registered. When calculated, this may still give what appears to be a high count rate but with a very large associated error (calculated conventionally as the square root of the total number of events counted from that sample).

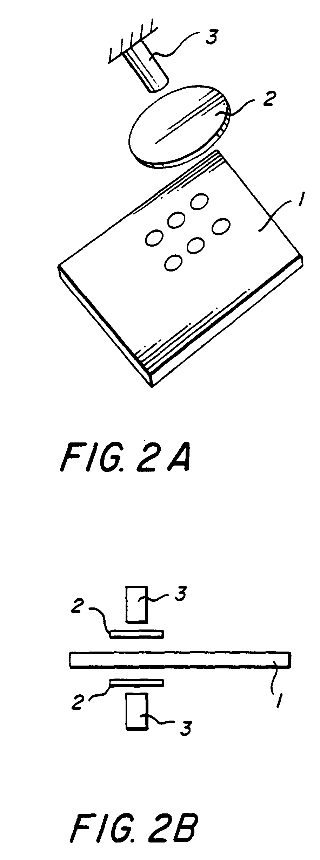

With the invention, this problem has been solved by placing a neutral density optical filtering means between the nylon and the PM tubes. A representation of the system of the invention appears in FIGS. 2A and 2B. As will be seen in that figure, a flat-bed scanning system is represented having a bed 1 and photodetector scanning means 3. The sample matrix under consideration will be placed on the bed 1 and chemiluminescent emissions from the sample will be detected by scanning means 3. The scanning means 3 may be located in a plurality of locations, including opposite sides of bed 3, as illustrated in FIG. 2B, as well as in multiple locations above or below bed 3. As a key element of this invention, optical filter means 2 is disposed between each scanning means 3 and the sample being observed by that scanning means.

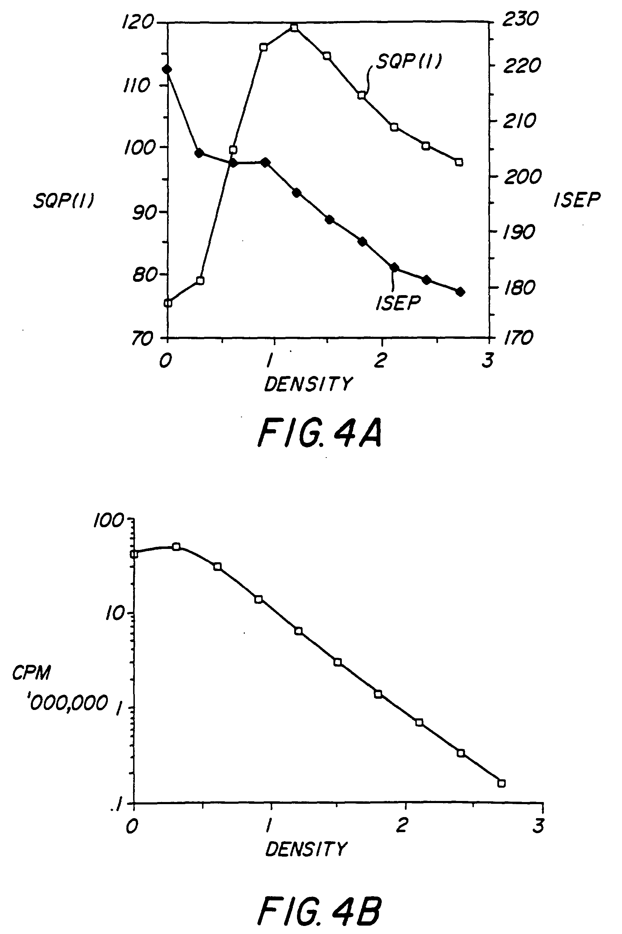

As shown in FIG. 4B, disposition of such a filter, using a neutral density filter, caused the count rate to initially increase slightly and then to decrease as filter density increased. This relationship can be explained by the fact that, without the neutral density filter in place, the coincidence of events results in an apparent merger of some of the pulses and thus will cause the counter to record a count rate less than the actual number of events. This is particularly the case where a large number of low energy events occur. These lower energy events are preferentially filtered out and the neutral density filter operates to achieve this result, thereby producing a unimodal pulse-height energy spectrum and the desired improvement in counting effectiveness.

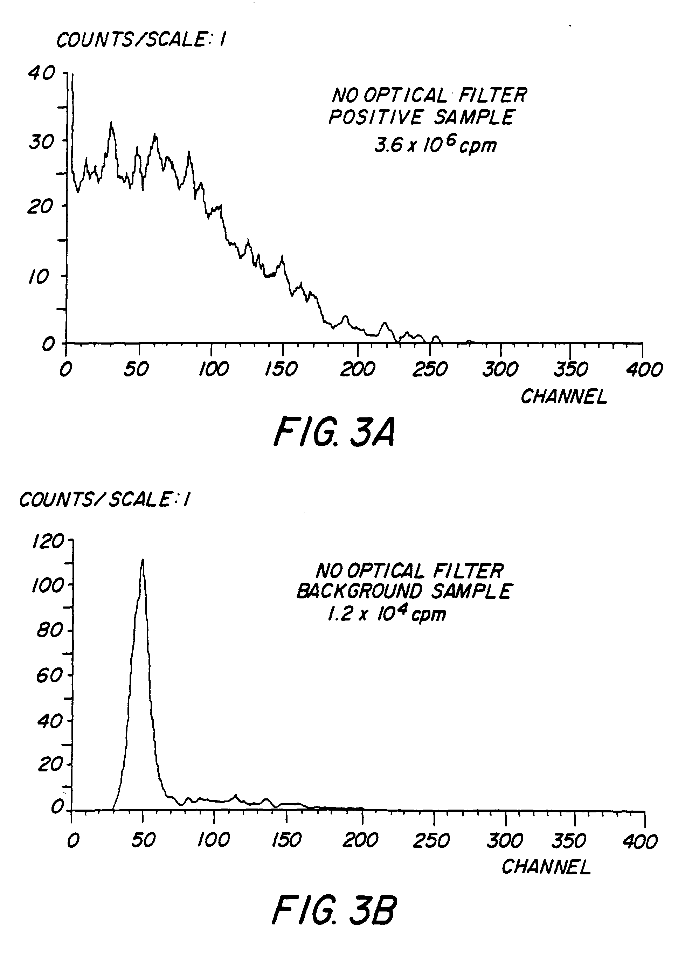

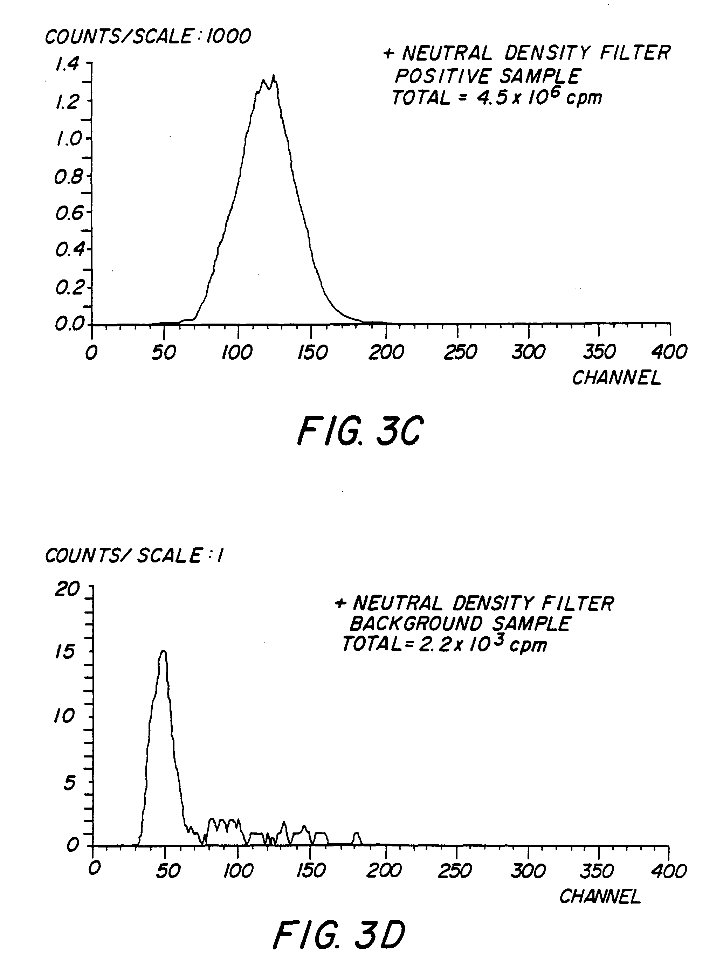

As will be seen in FIGS. 3A and 3C the pulse height spectrum changes with decreasing count rate. Without the filters a broad peak occurs in the pulse height spectrum, as seen in FIG. 3A. With the filters that peak becomes much sharper (FIG. 3C) and therefore the pulse height channels window can be reduced to channels 75-250 with the instrumentation of choice for this embodiment, as compared with channels 5-320 when set in coincidence mode for scintillation counting of tritium with this instrumentation. By comparing FIGS. 3B and 3D, it can be seen that the invention results in a reduction of the PM tube noise counts to about 1000/min for quartz photo-multiplier tubes and about 4000/min for a conventional tube machine and also results in an improved signal-to-noise ratio. In the described embodiment, chemiluminescence of both background signals were well above the instrumentation background levels and adequate precision was easily obtained with 10-30 second counting time.

FIG. 4A illustrates that the mean pulse height channel, or SQP(I), will increase to a maximum as the filter density is increased and then will be reduced if the density is increased further. Thus by looking at the position of the peak of the SQP(I) curve of FIG. 4A, one can choose a neutral density filter that gives a maximum mean energy level. In the described embodiment of the invention, a density of 1.2 optical density units was found to be optimum. However, a range of up to 1.8 optical density units was found to produce satisfactory results, especially where high pulse rates could result in reduced count rate due to merging of pulses.

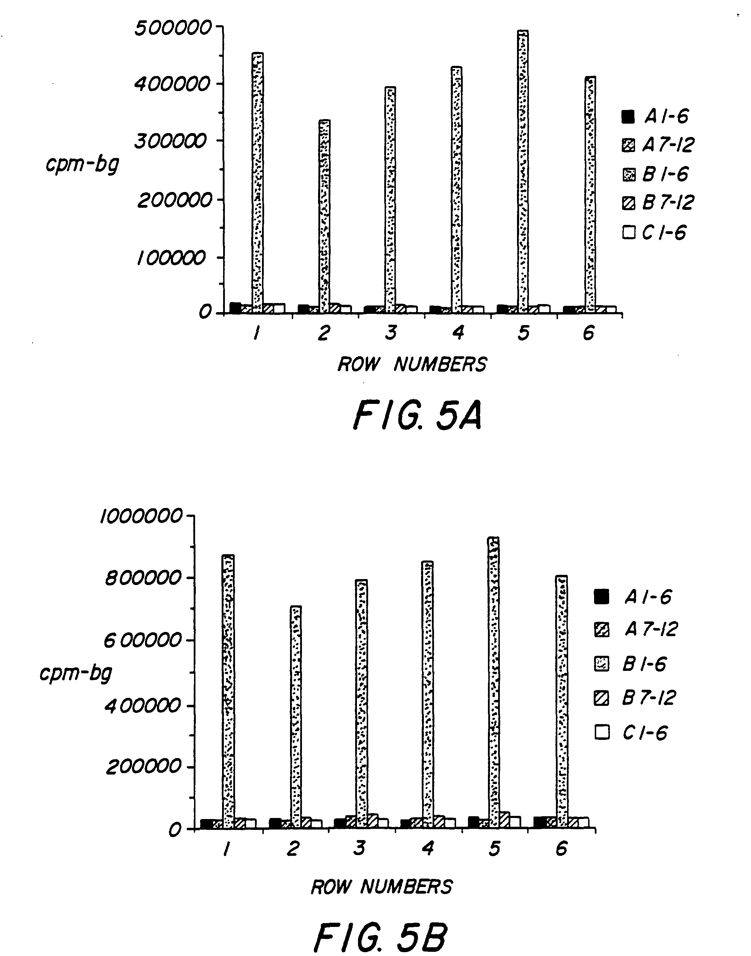

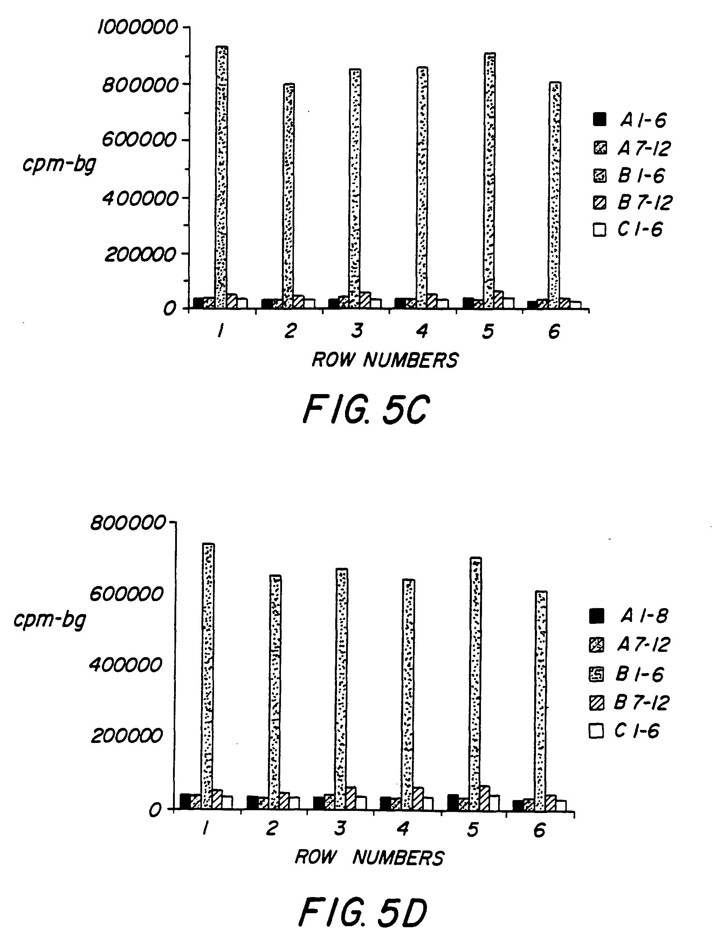

In the disclosed invention, cross talk -- defined as the counts detected in an adjacent blank sample area to an active sample - was not detected for chemiluminescent samples consisting of six test samples in a row which were measured together with the two blank rows on either side (FIG. 5A) with a large format Wallac Betaplate™ flat bed counter. With measurements made on days 2 - 5 (FIGS. 5B-D), some small amount of diffusion of sample or dissociation of probe gave rise to a low level of counts in adjacent samples areas although this was not cross talk. Cross talk was similarly not detected using a small 8 x 12 microtitre format in the Wallac Microbeta™ scintillation counter, also in non-coincident mode. In contrast, cross talk can be a serious problem in prior-art liquid flat-bed scintillation counting of filters, requiring, for example, the disposition of a black grid for absorption of the laterally emitted light, such as disclosed in US Patent No. 4,728,792 issued to Warner and Potter.

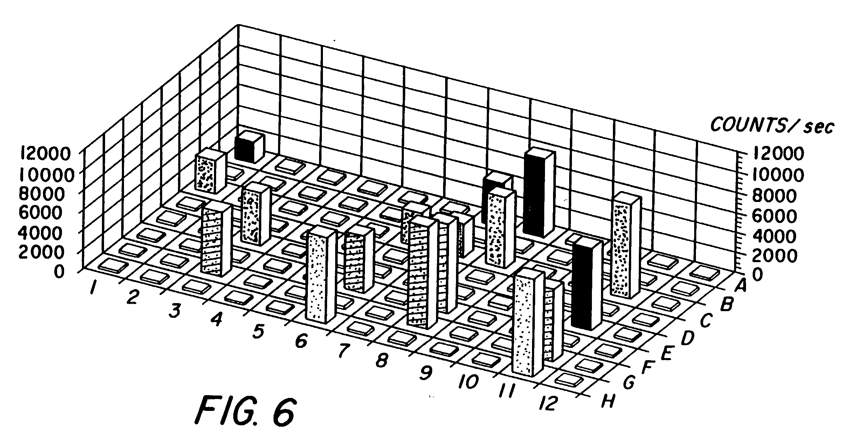

With the present invention, however, the chemiluminescent samples can be counted for 10-30 seconds with good precision, so that a 96 sample plate would take no more than 10 minutes to count on a 6-tube flat-bed counter with 30 sec/sample counting times. Typical results are shown in FIG. 6 where 5*µ*L DNA solution aliquots were spotted on nylon - - the samples being derived from PCR amplification of cDNA produced by the action of reverse transcriptase on mRNA. As will be readily understood, this counting methodology represents a much shorter procedure and is more convenient than autoradiography where, however short the exposure time, the filter must be placed next to the film in a dark-room and later removed and processed. In addition, the novel methodology gives a direct quantitative measure of the hybridization reaction.

It should also be observed that autoradiography could be accomplished for these samples with exposure of 10 minutes - 2 hours. By comparison, autoradiographic detection of probes labeled with ³²P will require exposure from overnight to several days. In addition, DNA dot blot samples labelled with ³²P may need up to 60 mins counting time.

Chemiluminescent samples detected according to the invention can also be used to detect bands in a series of tracks or lanes such as those produced by electrophoresis. And, the above described system can be used to scan the tracks in small increments thereby building up quantitative data to allow integration of counts for each band encountered during the scan. Samples arranged as a series of bands are scanned in one direction whereas samples arranged as spots whose positions are not known beforehand are scanned in two dimensions

For machines with double photo-multiplier tubes normally used in coincidence for scintillation counting, it will be possible to use both tubes independently, one each to count photons from samples on each of two supporting matrices placed back-to-back with a light proof barrier between them. A six-tube flat-bed counter (such as the Wallac Betaplate™ or Microbeta™ counter) could then count 12 samples at the same time for even more rapid throughput of samples.

As a further embodiment of the invention, more than one chemiluminescent system can be employed simultaneously. For example, one nucleic acid probe could be labelled with digoxygenin and a second probe labeled with biotin. The labels could then be detected with an antibody (or streptavidin for biotin) conjugated with a different enzyme. The DIG system from Boehringer uses alkaline phosphatase with AMPPD and has a maximum light output at a wavelength of 477nM, while the ECL system from Amersham uses horseradish peroxidase with its substrate of dioxetane and produces light at a maximum output at a wavelength of 428nM. As is true in this example case, the two (or more) systems must not interact chemically.

In this double-labeling embodiment, the difference in color spectrum output can be detected by the use of appropriate optical filters, successively interposed between the sample and photo-multipliers. Of course, the spectral windows of the filters for the top and bottom PM tubes must be chosen to pass the light at peak wavelength for the output of each assay. In addition, neutral density filters will reduce the output to levels suitable for counting.

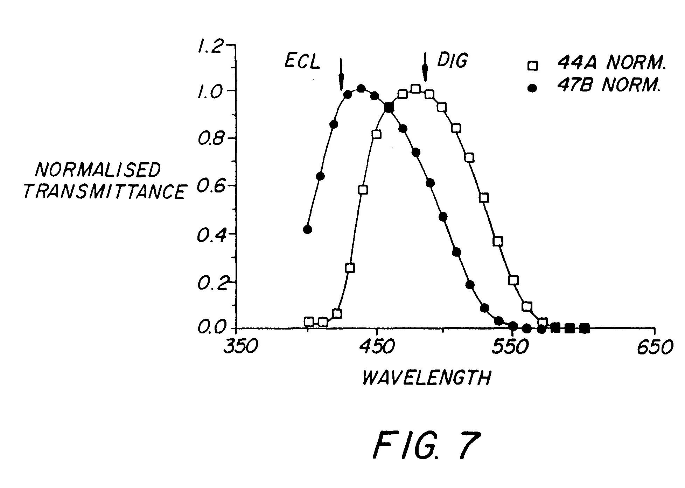

In a preferred embodiment, filters 44A and 47B from the Tricolor Filter range from Eastman Kodak would be used. Normalized transmittance data for these two filters is shown in FIG. 7 to illustrate the displaced peaking characteristics of those filters as related to the spectral output of the DIG and ECL treated probe.

The ratio of outputs of one optical filter compared with the other filter for singly labelled standardization samples would permit determination of calibration data. For mixed samples the ratio of outputs could be used in conjunction with the calibration data to calculate each probe's contribution to the count rate. It will be appreciated that this method represents an improvement compared with parallel sets of samples being tested successively. For example, one can test the exact same sample for two different sequences or types of molecule, making it possible, among other things, to include an internal control.

As a further refinement of this double-labeling embodiment, a pair of photo-multipliers, one on each side of the sample, can be used with different optical filters suitable for the respective light output. The ratio of the simultaneous count rates so obtained would allow double label counting with a high throughput.

Finally, if a pair of photo-multiplier tubes are used with one or two chemiluminescent systems being detected, it will also be possible to count samples labelled with radioactivity by recording the coincident events from both tubes -- although the chemiluminescence must be low enough for coincident single photon events to be rare compared with radioactive decay of the sample. Even if small rates of chemiluminescent coincidence are detected, this tends to have low pulse height, whereas for isotopes such as ³⁵S and ³²P used from DNA probe labelling, the mean pulse height is much higher than chemiluminescence and can be separated by appropriate setting of the pulse height window.

For dual radioactive/chemiluminescent labelling there must be minimal interaction under the different counting conditions required for the different measurements, or at least there should be a means of correction. To test the effectiveness of the invention in satisfying this criteria, a dilution series of chemiluminescent samples were first counted under conditions suitable for chemiluminescence and then samples with radioactive phosphorus-32, either alone or spotted on top of the chemiluminescent samples, were counted. For the radioactive measurement of phosphorus, the water-wet nylon filter wrapped in plastic film was sandwiched between two glass fibre filters each being within a thin plastic bag and permeated with scintillant. This method of measuring ³²P is described by C. G. Potter and S. Le Jeune, "Quantitative analysis of nucleic acid dot blots using the Betaplate flat-bed scintillation counter," (1991) Technique, 3, 117-121. For this application the Wallac Microbeta counter was set up for filter counting, including use of a neutral density filter of 1.8 density units - specifically our filter was comprised of 2 Lee 211 filters each of 0.9 density units. The pulse height spectrum for ³²P shown in FIG. 7 is similar to that shown by Potter and Le Jeune using their larger format Betaplate counter.

Table 1 shows that non-coincident count rates are decreased by the 1.8 density unit neutral density filters, although addition of glass fibre/scintillant filters increased the count rates slightly. This is believed to be due to better reflection of chemiluminescent photons by the backing glass fibre filter compared with the (dark) photo-multiplier assembly. The total of randomly coincident counts ranged from 3.03% at 8,380 non-coincident counts to 13.4% of 269,150 cpm, varying with the count rate as expected. If the energy channels were restricted to those suitable for ³²P (channels 201-900) in the instrumentation of choice, it was difficult to measure the counts, as they were less than 0.014% of the total non-coincident counts. Most coincidences were in channels 75-200. These channels are also used for the non-coincidence counting of chemiluminescence.

**Table 1.**

| **Counts per minute above background of chemiluminescent and** ^{**32**}**P labelled samples** | | | | |
|---|---|---|---|---|
| Chemiluminescent Samples - means | | 1.(n=3) | 2.(n=4) | 3.(n=4) |
| 1. | No coincidence but with glass fibre filters + scintillant. Pulse height channels 75-200. | 559760 | 160090 | 20670 |
| | | | | |
| 2. | Same as 1 above but also with neutral density filter. | 269150 | 57780 | 8380 |
| | | | | |
| 3. | Same as 2 above but without glass fibre filter sandwich. | 236010 | 46090 | 8970 |
| | | | | |
| 4. | Same as 1 above but with coincidence (% of 1 above random coincidence) | a. 35900 (13.3) | 4798 (8.3) | 254 (3.0) |
| | | | | |
| | Channels 201-900 for ³²P | b. 11.5 (0.03) | 7.5 (0.16) | 0.4 (0.15) |
| 5. | ³²P samples on nylon (n=3) with glass fibre filter sandwich only, plus coincidence | mean = 692 ± 27 S.D. | | |
| | | | | |
| 6. | Same as 5 above but channels 75-200 | mean = 0.0 ± 0.0 S.D. | | |
| | | | | |
| 7. | ³²P samples (n=8) on nylon with chemiluminescence-emitting background | mean = 729 ± 73 S.D. | | |
| | | | | |
| 8. | Same as 7 above but channels 75-200 | mean = 1218 ± 1144 S.D. | | |

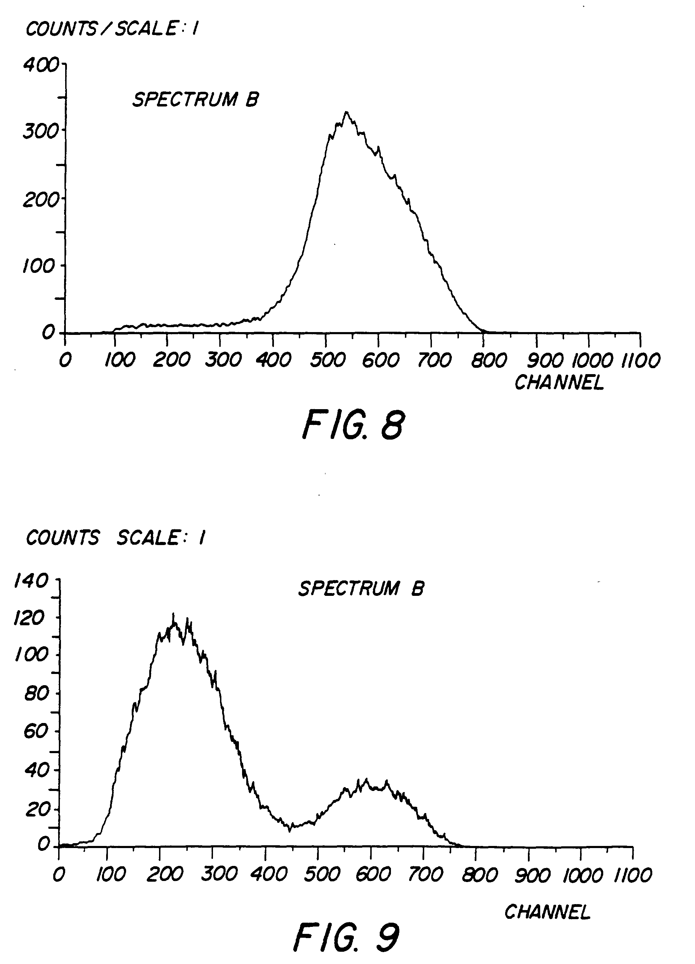

Three samples consisting of DNA probes labelled with ³²P were spotted onto nylon in amounts typical for hybridization assays as shown in Table 1. Very few counts were registered in channels 75-200 when these samples were counted in the non-coincidence long-pulse mode (Spectrum A on the machine), suitable for chemiluminescence. Because the neutral density filter cut down the light output, the pulse-height spectra were lower and bimodal as illustrated in FIG. 9. Overall counting efficiency for ³²P was reduced by about 60% in a subsequent test with higher activity samples because of the presence of the neutral density filter.

Samples already emitting chemiluminescence and previously measured were then spotted with ³²P radioactive probe at a low activity typical of hybridization samples and recounted. The instrument was then set to measure shorter pulses (Spectrum B on the machine), suitable for ³²P, and it was found that there was some interference of the random coincidences of the chemiluminescence with the phosphorus measured in channels 201-900. This spillover varied with chemiluminescent count rate as described above and this was more reliably determined from the long-pulse mode because the shorter pulse length mode gave rise to very high count rates (5-70 million cpm) that were difficult to manage as counting times were sometimes down to 0.03 sec.

The interference could be reduced by choosing a higher setting for the lower discriminator gate. For 8 samples placed on background chemiluminescence with 1000 cpm in channels 75-200 (Sample 7 of Table 1), there was no apparent interference as the mean cpm of the 8 samples was not significantly different from the mean of the three spotted on nylon.

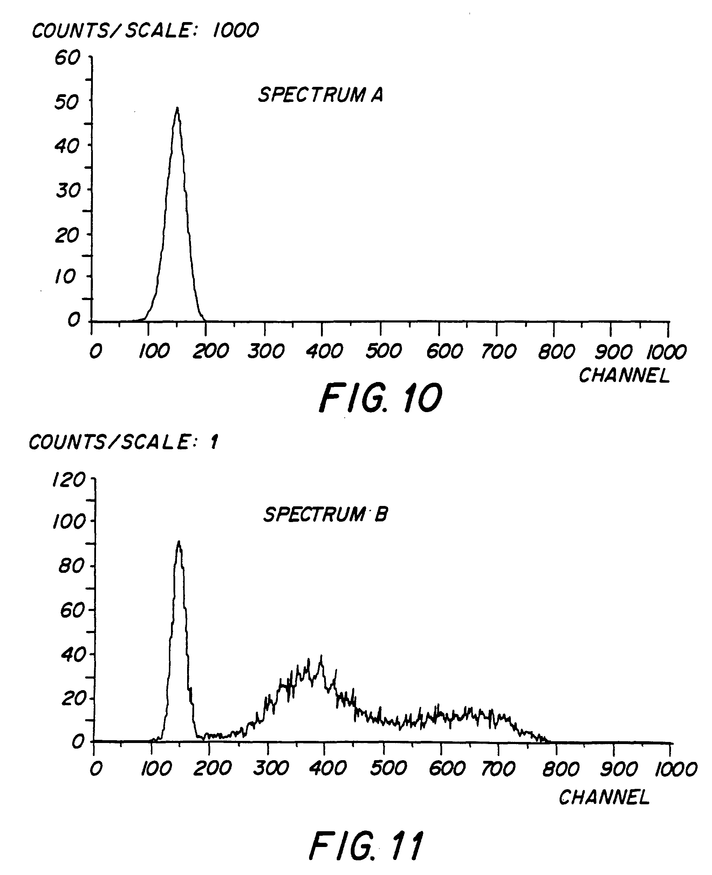

FIG. 10 shows the long-pulse non-coincidence pulse height spectrum for digoxigenin/AMPPD chemiluminescence alone. FIG. 11 shows coincident counting of shorter pulses (80-100 nS) for ³²P together with chemiluminescence. As before, the sample bearing filter was set up between a sandwich of glass fibre filters bagged with scintillant and neutral density filters of optical density 1.8 placed between the filter assembly and the active photo-multiplier tubes.

Thus, it is seen that a scintillant containing sandwich with neutral density filters can be used to measure simultaneously (or in succession): (1) chemiluminescence by short pulse counting with low energy pulse height discrimination and (2) radioactivity by counting shorter pulse lengths with higher pulse height discrimination. It may also be necessary to use a series of samples having different chemiluminescent activities to provide correction values for random coincidence spillover into coincidence counting.

Although the invention has been described in conjunction with a probe hybridization assay using a specific chemiluminescent system, it is to be understood that the invention can be used for performing any assay where chemiluminescence is used as a detection means. Such assays include immunoassays, receptor-ligand assays and the like. Furthermore, the invention can be used in conjunction with any known system for producing chemiluminescence.

The terminology "continuous support" and "supporting matrix" used herein are intended to include microtitration format filter matrices, such as a 96 well microtitration plate.

Herein, we have described a method of and apparatus for the very rapid quantification of chemiluminescent samples, that is much quicker than autoradiography or any known isotope labelling detection technique. This unique invention may become very important in high throughput research projects and diagnosis. Although the present embodiment of the invention has been described in detail, it should be understood that various changes, alterations and substitutions can be made therein without departing from the spirit and scope of the invention as defined by the appended claims.

## Claims

1. A method for performing a chemiluminescent assay comprising:
depositing a test sample on a supporting matrix (1);
effectuating chemiluminescence of the test sample;
filtering light radiating from the test sample as a result of said chemiluminescence; and
counting chemiluminescence from the filtered light;
characterised in that said filtration step comprises using a neutral density light attenuation optical filter (2) to remove low energy bursts of light giving rise to high count rates of low amplitude pulses.

2. The method of claim 1 wherein counting of luminescence is accomplished using a photodetector (3) equipped with a multichannel analyzer.

3. A method according to claim 1 wherein the chemiluminescent assay is performed on multiple test samples and the supporting matrix is a continuous supporting matrix (1), the method comprising the steps of:
depositing the multiple test samples on the continuous supporting matrix (1);
effectuating chemiluminenscence of the test samples;
filtering light radiating from each of the multiple test samples as a result of the chemiluminescence to remove said low energy bursts of light by said neutral density light attenuation optical filter (2); and
counting chemiluminescence of each of the samples from the filtered light.

4. The method of claim 3 wherein the multiple samples are disposed in a multiwell plate (1)

5. The method of claim 3 wherein chemiluminescence is counted using a multidetector system equipped with multichannel analyzers.

6. The method of claim 3 wherein chemiluminescence is counted using a scintillation counter operating in a non-coincidence mode.

7. The method of performing chemiluminescence assay as recited in claim 3, wherein said filtering step comprises the step of filtering the radiated light so that only a unimodal pulse height energy spectrum is produced during said counting step. method of performing chemiluminescence assay as recited in

8. The method of performing chemiluminescence assay as recited in claim 3, wherein the multiple test samples are nucleic acid samples bound to said continuous supporting matrix (1), said supporting matrix (1) is a nylon membrane and said reference sample is a DNA probe or RNA probe.

9. The method of performing chemiluminescence assay of claim 3, wherein said chemiluminescence assay is an immunoassay.

10. The method of performing chemiluminescence assay of claim 3, wherein said chemiluminescence assay is a receptor-ligand assay.

11. The method of performing chemiluminescence assay of claim 3, wherein said chemiluminescence assay is a DNA hybridization assay or RNA hybridization assay.

12. An apparatus for performing a chemiluminescence assay on multiple test samples deposited together on a continuous support (1), the continuous support (1) containing a reactant that effectuates chemiluminescence of the multiple test samples, the apparatus comprising:
flat bed means for holding the multiple test samples deposited on the continuous support (1) during the performance of the assay;
light detector means (3) for detecting light emitted during chemiluminescence of the multiple test samples, said light detector means being disposed opposite said flat bed;
chemiluminescence counter means for counting chemiluminescence of the multiple test samples, said chemiluminescence counter means coupled to said light detector means (3); and
filter means (2) for filtering light emitted during chemiluminescence and passing the light to said light detector means, said filter means (2) being disposed in the optical path between said flat bed means and said light detector means;
characterised in that said filter means (2) is a neutral density light attenuation optical filter (2) that precludes low energy bursts of light giving rise to high count rates of low amplitude pulses from being detectable by said light detector means (3).

13. The apparatus for performing chemiluminescent assay as recited in claim 12, wherein the multiple samples are disposed in a multiwell plate (1).

14. The apparatus for performing chemiluminescent assay as recited in claim 12, wherein said continuous support (1) comprises two supports placed back-to-back with a light proof barrier between them.

15. The apparatus for performing a chemiluminescent assay as recited in claim 12, wherein the density of said neutral density filter (2) is from about 1.2 to about 1.8 optical density units.

16. The apparatus for performing chemiluminescent assay as recited in claim 12, wherein said light detector means (3) is at least one photomultiplier tube of a multi-channel analyzer, and said chemiluminescence counter is a plurality of counter registers for storing the number of chemiluminescent events detected.

17. The apparatus for performing chemiluminescent assay as recited in claim 12, wherein the multiple chemiluminescent samples are bound to a continuous support (1) in the form of an M x N array, wherein M and N are positive integers.

18. The apparatus for performing chemiluminescent assay as recited in claim 17, wherein a pair of said filter is provided and wherein said light detector means comprises a pair of photomultipliers wherein two labelled nucleic acid probes, one comprising a chemiluminescent probe and the other a radioactive probe are detected by respective pairs of photomultipliers (3) and optical filters (2), the pairs being disposed on opposite sides of the sample and the optical filters (2) being different.

19. A method of conducting electrophoresis comprising the steps of:
depositing multiple test samples on a continuous supporting matrix (1);
conducting gel electrophoresis;
effectuating chemiluminescence of the test samples for a suitable exposure time;
filtering light radiating from the multiple test samples as a result of the chemiluminescence with a neutral density filter (2); and
counting chemiluminescence from the filtered light;
characterised in that said filtration step comprises using a neutral density light attenuation optical filter (2) to remove low energy bursts of light giving rise to high count rates of low amplitude pulses.

20. The method of claim 19 wherein, following conducting of gel electrophoresis, the test samples are transferred to a transfer membrane.

21. The method of claim 20 wherein the transfer membrane is nylon.

## Patentansprüche

1. Verfahren zur Durchführung von Chemilumineszenzassays, beinhaltend das Bringen einer Versuchsprobe auf eine Trägermatrix (1) ; das Bewirken einer Chemilumineszenz der Versuchsprobe; das Filtern des Lichts, das als Ergebnis der Chemilumineszenz von der Versuchsprobe ausgesandt wird, und das Zählen der Chemilumineszenzen in dem gefilterten Licht, dadurch gekennzeichnet, dass der Filterschritt die Verwendung eines neutraldichten optischen Lichtschwächungsfilters (2) umfasst, so dass niederenergetischer Lichterscheinungen, die hohe Zählraten niederamplituder Pulse ergeben, beseitigt werden.

2. Verfahren nach Anspruch 1, wobei das Zählen der Chemilumineszenz mit einem Photodetektor (3) erfolgt, der mit einem Mehrkanalanalysator ausgerüstet ist.

3. Verfahren nach Anspruch 1, wobei der Chemilumineszenzassay mit mehreren Versuchsproben erfolgt und die Trägermatrix eine kontinuierliche Trägermatrix (1) ist, wobei das Verfahren die Schritte umfasst:
Bringen mehrerer Versuchsproben auf die kontinuierliche Trägermatrix (1) ;
Bewirken einer Chemilumineszenz der Versuchsproben;
Filtern des Lichts, das jeweils als Ergebnis der Chemilumineszenz von den Versuchsproben ausgesandt wird, und
Entfernen der niederenergetischen Lichterscheinungen mit einem neutraldichten optischen Lichtschwächungsfilter (2), und
Zählen der Chemilumineszenzen einer jeden Probe in dem gefilterten Licht.

4. Verfahren nach Anspruch 3, wobei die mehreren Proben auf einer Multiwell-Platte (1) angeordnet sind.

5. Verfahren nach Anspruch 3, wobei die Chemilumineszenz mit einem Multidetektorsystem, das mit einem Mehrkanalanalysator ausgerüstet ist, gezählt wird.

6. Verfahren nach Anspruch 3, wobei die Chemilumineszenz mit einem Szintillationszähler, der im Nichtkoinzidenzmodus arbeitet, gezählt wird.

7. Verfahren zur Durchführung eines Chemilumineszenzassays nach Anspruch 3, wobei der Filterschritt beinhaltet das Filtern des ausgestrahlten Lichts, so dass beim Zählschritt nur unimodale Pulshöhen im Energiespektrum erzeugt werden.

8. Verfahren zur Durchführung eines Chemilumineszenzassays nach Anspruch 3, wobei die mehreren Versuchsproben Nucleinsäureproben sind, die an eine kontinuierlichen Trägermatrix (1) gebunden sind, wobei die Trägermatrix
(1) eine Nylonmembran ist und die Bezugsprobe eine DNA- oder RNA-Probe.

9. Verfahren zur Durchführung eines Chemilumineszenzassays nach Anspruch 3, wobei der Chemilumineszenzassay ein Immunoassay ist.

10. Verfahren zur Durchführung eines Chemilumineszenzassays nach Anspruch 3, wobei der Chemilumineszenzassay ein Rezeptor-Liganden-Assay ist.

11. Verfahren zur Durchführung eines Chemilumineszenzassays nach Anspruch 3, wobei der Chemilumineszenzassay ein DNA-Hybridisierungsassay oder ein RNA-Hybridisierungsassay ist.

12. Vorrichtung zur Durchführung eines Chemilumineszenzassays an mehreren Versuchsproben, die zusammen auf einen kontinuierlichen Träger (1) gebracht wurden, wobei der kontinuierliche Träger (1) einen Reaktanden aufweist, der eine Chemilumineszenz der mehreren Versuchsproben bewirkt, wobei die Vorrichtung beinhaltet:
eine Flachbetteinrichtung zum Halten der mehreren Versuchsproben, die bei der Durchführung des Assays auf dem kontinuierlichen Träger (1) abgelegt sind;
eine Lichtdetektoreinrichtung (3) zum Erfassen des bei der Chemilumineszenz von mehreren Versuchsproben ausgestrahlten Lichts, wobei die Lichtdetektoreinrichtung dem Flachbett gegenüberliegend angeordnet ist;
eine Chemilumineszenz-Zähleinrichtung zum Zählen der Chemilumineszenzen der mehreren Versuchsproben, wobei die Chemilumineszenz-Zähleinrichtung mit der Lichtdetektoreinrichtung (3) gekoppelt ist, und
eine Filtereinrichtung (2) zum Filtern des bei der Chemielumineszenz ausgestrahlten Lichts und Leiten des Lichts zu der Lichtdetektoreinrichtung, wobei die Filtereinrichtung (2) im optischen Weg zwischen der Flachbetteinrichtung und der Lichtdetektoreinrichtung angeordnet ist; dadurch gekennzeichnet,
dass die Filtereinrichtung (2) ein neutraldichter optischer Lichtschwächungsfilter (2) ist, der niederenergetische Lichterscheinungen, welche hohe Zählraten niederamplituder Pulse ergeben, von der Erfassung durch die Lichtdetektoreinrichtung (3) ausschließt.

13. Vorrichtung zur Durchführung eines Chemilumineszenzassays nach Anspruch 12, wobei die mehreren Proben in einer Multiwell-Platte (1) angeordnet sind.

14. Vorrichtung zur Durchführung eines Chemilumineszenzassays nach Anspruch 12, wobei der kontinuierliche Träger (1) zwei Träger beinhaltet, die Rücken an Rücken mit einer lichtdichten Trennung dazwischen angeordnet sind.

15. Vorrichtung zur Durchführung eines Chemilumineszenzassays nach Anspruch 12, wobei der Neutralfilter (2) eine Dichte von etwa 1,2 bis etwa 1,8 optischen Dichteeinheiten hat.

16. Vorrichtung zur Durchführung eines Chemilumineszenzassays nach Anspruch 12, wobei die Lichtdetektoreinrichtung (3) mindestens eine Photomultiplierröhre eines Multikanalanalysators ist, und der ChemilumineszenzZähler zum Speichern der Zahl der erfassten Chemilumineszenzereignisse mehrere Zählregister besitzt.

17. Vorrichtung zur Durchführung eines Chemilumineszenzassays nach Anspruch 12, wobei die mehreren chemilumineszenten Proben an einen kontinuierlichen Träger (1) in Form einer M^{x}N-Anordnung gebunden sind, wobei M und N ganze Zahlen sind.

18. Vorrichtung zur Durchführung eines Chemilumineszenzassays nach Anspruch 17, in der ein Paar der Filter vorgesehen ist und die Lichtdetektoreinrichtungen ein Photomultiplierpaar aufweist, wobei zwei markierte Nucleinsäureproben, wovon eine eine chemilumineszenten Probe umfasst und die andere radioaktiv ist, erfasst werden von zughörigen Paaren von Fotomultipliern (3) und optischen Filtern (2), wobei die Paare an den gegenüberliegenden Seiten der Probe angeordnet sind und die optischen Filter (2) verschieden sind.

19. Verfahren zur Durchführung einer Elektrophorese, beinhaltend die Schritte:
das Bringen von mehreren Versuchsproben auf eine kontinuierlichen Trägermatrix (1) ;
das Durchführen der Gelelektrophorese;
das Bewirken einer Chemilumineszenz der Versuchsproben für eine geeignete Belichtungszeit;
das Filtern des als Ergebnis der Chemilumineszenz von den mehreren Versuchsproben ausgesandten Lichts mit einem Neutralfilter (2); und
das Zählen der Chemilumineszenzen in dem gefilterten Licht;
dadurch gekennzeichnet, dass der Filterschritt beinhaltet die Verwendung eines neutraldichten optischen Lichtschwächungsfilters (2) zur Entfernung der niederenergetischen Lichterscheinungen, welche hohe Zählerraten niederamplituder Pulsen ergeben.

20. Verfahren nach Anpruch 19, wobei nach der Durchführung der Gelelektrophorese die Versuchsproben auf eine Übertragungsmembran gebracht werden.

21. Verfahren nach Anspruch 20, wobei die Übertragungsmembran Nylon ist.

## Revendications

1. Procédé d'exécution d'une analyse par chimiluminescence, comprenant :
le dépôt d'un échantillon sur une matrice de support (1),
l'exécution d'une chimiluminescence de l'échantillon,
le filtrage de la lumière provenant de l'échantillon à la suite de la luminescence, et
le comptage de la chimiluminescence à partir de la lumière filtrée,
caractérisé en ce que l'étape de filtrage comprend l'utilisation d'un filtre neutre d'atténuation de lumière (2) destiné à retirer les salves de faible énergie de la lumière donnant des taux élevés de comptage d'impulsions à faible amplitude.

2. Procédé selon la revendication 1, dans lequel le comptage de la luminescence est réalisé à l'aide d'un photodétecteur (3) équipé d'un analyseur à plusieurs canaux.

3. Procédé selon la revendication 1, dans lequel l'analyse par la chimiluminescence est réalisée sur plusieurs échantillons et la matrice de support est une matrice continue de support (1), le procédé comprenant les étapes suivantes :
le dépôt des multiples échantillons sur la matrice continue de support (1),
l'exécution de la chimiluminescence des échantillons,
le filtrage de la lumière provenant de chacun des multiples échantillons à la suite de la chimiluminescence afin que les salves de lumière de faible énergie soient retirées par le filtre optique neutre (2) d'atténuation de lumière, et
le comptage de la chimiluminescence de chacun des échantillons à partir de la lumière filtrée.

4. Procédé selon la revendication 3, dans lequel les multiples échantillons sont placés sur une plaque (1) à plusieurs cellules.

5. Procédé selon la revendication 3, dans lequel la chimiluminescence est comptée à l'aide d'un système à plusieurs détecteurs équipé d'analyseurs à plusieurs canaux.

6. Procédé selon la revendication 3, dans lequel la chimiluminescence est comptée à l'aide d'un compteur à scintillation travaillant en mode sans coïncidence.

7. Procédé d'exécution d'une analyse par chimiluminescence selon la revendication 3, dans lequel l'étape de filtrage comprend le filtrage de la lumière émise afin que seul un spectre d'énergie unimodale de hauteur d'impulsion soit produit pendant l'étape de comptage.

8. Procédé d'exécution d'une analyse par chimiluminescence selon la revendication 3, dans lequel les multiples échantillons sont des échantillons d'acide nucléique liés à la matrice continue de support (1), la matrice de support
(1) est une membrane de " Nylon", et l'échantillon de référence est un vecteur de ADN ou un vecteur de ARN.

9. Procédé d'exécution d'une analyse par chimiluminescence selon la revendication 3, dans lequel l'analyse par chimiluminescence est une analyse immunologique.

10. Procédé d'exécution d'une analyse par chimiluminescence selon la revendication 3, dans lequel l'analyse par chimiluminescence est une analyse de récepteur-ligand.

11. Procédé d'analyse par chimiluminescence selon la revendication 3, dans lequel l'analyse par chimiluminescence est une analyse par hybridation de ADN ou de ARN.

12. Appareil d'analyse par chimiluminescence de multiples échantillons déposés ensemble sur un support continu (1), le support continu (1) contenant un réactif qui provoque une chimiluminescence des multiples échantillons, l'appareil comprenant :
un dispositif à support plat destiné à supporter les multiples échantillons déposés sur le support continu (1) au cours de l'exécution de l'analyse,
un dispositif détecteur de lumière (3) destiné à détecter la lumière émise par chimiluminescence des multiples échantillons, le dispositif détecteur de lumière étant placé en face du support plat,
un dispositif compteur de chimiluminescence destiné à compter la chimiluminescence des multiples échantillons, le dispositif compteur de chimiluminescence étant couplé au dispositif détecteur de lumière (3), et
un dispositif (2) à filtre de filtrage de la lumière émise par chimiluminescence et de transmission de la lumière au dispositif détecteur de lumière, le dispositif à filtre
(2) étant placé sur le trajet optique entre le dispositif à support plat et le dispositif détecteur de lumière,
caractérisé en ce que le dispositif à filtre (2) est un filtre optique neutre (2) d'atténuation de lumière qui empêche la détection des salves de lumière de faible énergie donnant des taux élevés de comptage d'impulsions de faible amplitude par le dispositif détecteur de lumière (3).

13. Appareil d'analyse par chimiluminescence selon la revendication 12, dans lequel les multiples échantillons sont disposés dans une plaque (1) à plusieurs cellules.

14. Appareil d'analyse par chimiluminescence selon la revendication 12, dans lequel le support continu (1) comporte deux supports placés dos à dos avec un organe de barrage arrêtant la lumière entre eux.

15. Appareil d'analyse par chimiluminescence selon la revendication 12, dans lequel la densité du filtre neutre (2) est comprise entre environ 1,2 et 1,8 unité de densité optique.

16. Appareil d'analyse par chimiluminescence selon la revendication 12, dans lequel le dispositif détecteur de lumière (3) est au moins un tube photomultiplicateur d'un analyseur à plusieurs canaux, et le compteur de chimiluminescence est formé de plusieurs registres compteurs destinés à conserver le nombre d'événements chimiluminescents détectés.

17. Appareil d'analyse par chimiluminescence selon la revendication 12, dans lequel les multiples échantillons chimiluminescents sont liés à un support continu (1) sous forme d'un arrangement M x N, M et N étant des nombres entiers positifs.

18. Appareil d'analyse par chimiluminescence selon la revendication 17, dans lequel une paire de filtres est utilisée, le dispositif détecteur de lumière comprend une paire de photomultiplicateurs, et deux vecteurs d'acide nucléique marqués, l'un comprenant un vecteur chimiluminescent et l'autre un vecteur radioactif, sont détectés par des paires respectives de photomultiplicateurs (3) et de filtres optiques (2), les paires étant disposées de part et d'autre de l'échantillon et les filtres optiques (2) étant différents.

19. Procédé d'exécution d'une électrophorèse, comprenant les étapes suivantes :
le dépôt de multiples échantillons sur une matrice continue de support (1),
l'exécution d'une électrophorèse sur gel,
l'exécution de la chimiluminescence des échantillons pendant un temps convenable d'exposition,
le filtrage de la lumière provenant des multiples échantillons à la suite de la chimiluminescence à l'aide d'un filtre neutre (2), et
le comptage de la chimiluminescence de la lumière filtrée,
caractérisé en ce que l'étape de filtrage comprend l'utilisation d'un filtre optique neutre (2) d'atténuation de lumière destiné à retirer les salves de lumière de faible énergie donnant lieu à des fréquences élevées de comptage d'impulsions de faible amplitude.

20. Procédé selon la revendication 19, dans lequel, après l'exécution de l'électrophorèse sur gel, les échantillons sont transférés à une membrane de transfert.

21. Procédé selon la revendication 20, dans lequel la membrane de transfert est formée de "Nylon".
